# EUROPÄISCHE PATENTSCHRIFT

(11) **EP 2 482 861 B1**
(45) Veröffentlichungstag und Bekanntmachung des Hinweises auf die Patenterteilung: **07.09.2016**
(21) Anmeldenummer: 10763320.8
(22) Anmeldetag: 30.09.2010
(51) Int. Cl.: A61L 24/00, A61L 24/02, A61L 24/06

(54) **KNOCHENZEMENT UND VERFAHREN ZU DESSEN HERSTELLUNG**
BONE CEMENT AND A METHOD FOR PRODUCING SAME
CIMENT OSSEUX ET PROCÉDÉ DE PRODUCTION ASSOCIÉ

(30) Priorität: 30.09.2009 DE 102009043550
(43) Veröffentlichungstag der Anmeldung: 08.08.2012
(73) Patentinhaber: AAP Biomaterials GmbH, 64807 Dieburg (DE)
(72) Erfinder: SATTIG, Christoph, 64807 Dieburg (DE); DINGELDEIN, Elvira, 64287 Darmstadt (DE)
(74) Vertreter: Herden, Andreas F.
(86) Internationale Anmeldenummer: PCT/EP2010/005951
(87) Internationale Veröffentlichungsnummer: WO 2011/038905

(56) Entgegenhaltungen:
- US-A- 4 093 576
- BERUTO D T; MEZZASALMA S A; CAPURRO M; BOTTER R; CIRILLO P: "Use of [alpha]-tricalcium phosphate (TCP) as powders and as an aqueous dispersion to modify processing, microstructure, and mechanical properties of polymethylmethacrylate (PMMA) bone cements and to produce bone-substitute compounds.", JOURNAL OF BIOMEDICAL MATERIALS RESEARCH, Bd. 49, Nr. 4, 15. März 2000 (2000-03-15), Seiten 498-505, XP002635061, ISSN: 0021-9304

## Beschreibung

Die Erfindung betrifft einen Knochenzement sowie ein Verfahren zu dessen Herstellung.

### Hintergrund der Erfindung

Aus der Praxis bekannt sind verschiedene Arten von Knochenzement. Um einen biodegradierbaren Knochenzement bereitzustellen, also einen Knochenzement, der nach dem Einsetzen im Laufe der Zeit durch nachwachsendes Knochengewebe ersetzt wird, sind beispielsweise Calciumphosphat- oder Calciumsulfat-basierte Knochenzemente bekannt. Je nach Anwendung kann mit diesen Knochenzementen eine hinreichende initiale Stabilität erreicht werden. Allerdings ist das ausgehärtete Material recht spröde, so dass für viele Anwendungsfälle keine hinreichende Dauerstabilität erreicht werden kann. Weiter sind Calciumphosphate bekannt. Bei Calciumphosphat-basierten Knochenzementen kommt es allerdings bereits nach kurzer Zeit zu einer Erweichung des Materials, so dass die Defektstelle in vielen Fällen, insbesondere bei älteren Patienten, nicht ausreichend schnell stabilisiert werden kann.

Calciumsulfat- oder Calciumphosphat-basierte Knochenzemente sind generell nicht zur Verankerung von dynamisch lasttragenden Implantaten geeignet.

Für diese Anwendungen werden generell, also nicht nur bei älteren Patienten, Acryl-basierte Knochenzemente verwendet. Diese haben den Vorteil einer hohen Steifigkeit bereits nach kurzer Aushärtung. Um den implantierten Acryl-basierten Zement bildet sich zumeist eine bindegewebeartige Schicht, es wächst kein Knochenmaterial in den Knochenzement ein, wobei ein Acryl-basierter Knochenzement in der Regel recht gut toleriert wird.

Beruto et.al. (JOURNAL OF BIOMEDICAL MATERIALS RESEARCH, Bd. 49, Nr. 4, 15. März 2000 (2000-03-15)) offenbart die Verwendung einer Alpha-Tricalciumphosphat Partikeln enthaltenden Suspension in einem PMMA-basierten Knochenzement.

US 4093576 offenbart ein Knochenzement, umfassend eine polymerisierbare hydrophobe Komponente enthaltend ein Acrylatmonomer (Methylmethacrylat), ein polymerisches Knochenzementpulver (PMMA Pulver) und eine hydrophile Komponente, umfassend ein wässriges Gel, das Carboxymethylcellulose-Pulver enthält.

Generell sind Acryl-basierte Knochenzemente nicht vom Körper abbaubar. Es wurde daher versucht, die Biokompatibilität derartiger Zemente durch Zusatz beispielsweise von Hydroxylapatit zu verbessern. Derartige Zuschläge sind zumeist allerdings nur an der Oberfläche des Zementes zugänglich, überwiegend von Acryl umhüllt und überwiegend nicht resorbierbar. Letztendlich kann daher der bekannte Zusatz von Calciumverbindungen in der Regel nur das Aufwachsen von Gewebe auf den Knochenzement verbessern, die Menge an zugänglichen Partikeln reicht aber in der Regel nicht aus, um eine Neubildung von Knochen im direkten Kontakt zu erreichen oder zu fördern. Weiter wird die Meinung vertreten, dass insbesondere bei vertebroplastischen und kyphoplastischen Anwendungen der Zusatz von Calciumverbindungen Nachteile mit sich bringt, da das E-Modul aufgrund der zugesetzten Partikel erhöht wird, was sich negativ auf den angrenzenden Knochen, insbesondere auf die angrenzenden Wirbelkörper auswirken kann. Die ohnehin bestehende Gefahr einer Frakturierung bei der Verwendung von relativ festen Acryl-basierten Knochenzementen kann daher mit dem Zusatz von Hydroxylapatit- Partikeln möglicherweise sogar steigen.

### Aufgabe der Erfindung

Die Erfindung betrifft demgegenüber einen Acryl-basierten Knochenzement, bei welchem die genannten Nachteile von bekannten Acryl-basierten Knochenzementen reduziert werden sollen.

Es ist insbesondere eine Aufgabe der Erfindung, einen Knochenzement bereitzustellen, welcher zumindest teilweise biodegradierbar ist und so von natürlichem Knochengewebe durchwachsen werden kann. Des Weiteren soll der Knochenzement in seiner Festigkeit besser an die Festigkeit des natürlichen Knochens angepasst werden können, wodurch insbesondere die Gefahr von Frakturen im Wirbelbereich reduziert werden soll.

### Zusammenfassung der Erfindung

Die Aufgabe der Erfindung wird bereits durch einen Knochenzement, einen ausgehärteten Knochenzement sowie durch ein Verfahren zum Anmischen von Knochenzement nach einem der unabhängigen Ansprüche gelöst.

Bevorzugte Ausführungsformen und Weiterbildungen der Erfindung sind den jeweiligen Unteransprüchen zu entnehmen.

Die Erfindung betrifft einen Knochenzement, welcher insbesondere zum Einsetzen von Prothesen, aber je nach Indikation auch zum Schließen oder Auffüllen von Knochendefekten vorgesehen ist.

Der Knochenzement umfasst zumindest eine polymerisierbare hydrophobe Komponente, zumindest eine hydrophile Komponente und biodegradierbares Material, welches biodegradierbare Partikel umfasst, wobei sich hydrophile und hydrophobe Komponente sich nicht mischen und wobei die biodegradierbaren Partikel zumindest teilweise in der hydrophilen Komponente enthalten sind, dadurch gekennzeichnet, dass hydrophile Komponente zusammen mit dem biodegradierbaren Material als Paste ausgebildet ist und wobei das biodegradierbare Material in nanopartikulärer Form vorliegt.

Unter einer hydrophilen und einer hydrophoben Komponente werden zwei Komponenten verstanden, welche sich aufgrund ihrer hydrophilen bzw. hydrophoben Eigenschaften nicht mischen. Die hydrophile Komponente umfasst vorzugsweise Wasser, wobei die biodegradierbaren Partikel eine Suspension im Wasser bilden. Beim Anmischen des Knochenzementes mit einem geeigneten Rührwerkzeug mischt sich die wässrige, hydrophile Komponente nicht mit der hydrophoben Komponente, so dass die biodegradierbaren Partikel in den durch die hydrophile Komponente gebildeten Poren zurückbleiben. Im Unterschied zum Zusatz von biodegradierbaren Partikeln als reine Zuschlagstoffe zur Polymermasse sind so die biodegradierbaren Partikel frei zugänglich. Es versteht sich, dass im Sinne der Erfindung unter "nicht mischbar" nicht gemeint ist, dass nicht auch ein Teil der hydrophilen Komponente bzw. ein Teil des Wassers sich in der hydrophoben Komponente, insbesondere in dem Polymer lösen kann. So nehmen bestimmte Acrylate bis zu 10 % Wasser auf. Bei geeignet dimensionierter Menge an Wasser ist aber sicher gestellt, dass noch immer genug Wasser als hydrophile Komponente ist, welches sich nicht mit dem Polymer mischt.

Bei einer bevorzugten Ausführungsform der Erfindung sind zumindest 50, vorzugsweise zumindest 80 % der biodegradierbaren Partikel in der hydrophilen Komponente enthalten. Ein großer Teil der Partikel ist so frei zugänglich. Im Unterschied zu bekannten Knochenzementen mit biodegradierbaren Partikel als Zuschlagstoff sind die Partikel nicht durch die Polymermatrix versiegelt.

Die hydrophobe Komponente umfasst insbesondere ein Acrylatmonomer, wobei unter einem Acrylatmonomer eine Verbindung verstanden wird, die zu einem Polyacryl polymerisierbar ist. Es handelt sich mithin um einen Acryl-basierten Knochenzement. Beispielsweise kann es sich um einen Acrylsäureester, insbesondere um Metylmethacrylat handeln. Mono- beziehungsweise Oligomere, aus denen sich Polyacrylate bilden, sind hinreichend bekannt und sind, zumindest im nicht ausgehärteten Zustand derart hydrophob, dass es in Verbindung mit Wasser in der Regel nicht zu Dispersionen mit einer feinen Verteilung kommt. Im ausgehärteten Zustand reduzieren Acrylat-basierte Knochenzemente in der Regel ihre hydrophoben Eigenschaften und können sogar Wasser bis zu einigen Prozent ihres Gewichtes aufnehmen. Gemäß der Erfindung müssen die hydrophoben Eigenschaften nur im nicht ausgehärteten Zustand vorliegen, damit sich nicht eine Lösung oder eine feine Dispersion aus den beiden Komponenten bildet.

Vielmehr bilden sich durch die hydrophile Komponente zumindest teilweise offenporige Bereiche, welche zunächst mit der hydrophilen Komponente ausgefüllt sind. Die biodegradierbaren Partikel, welche vorzugsweise in der hydrophilen Komponente enthalten sind, lagern sich überwiegend in den Poren an, so dass nach dem Aushärten ein zumindest teilweise offenporig ausgehärteter Acrylzement vorliegt, dessen Poren mit biodegradierbarem Material besetzt sind.

Aufgrund der offenen Poren kann natürliches Knochengewebe in den ausgehärteten Knochenzement einwachsen, wobei das Einwachsen durch die biodegradierbaren Partikel erheblich beschleunigt wird.

Die Erfinder konnten somit einen Acryl-basierten Knochenzement bereitstellen, welcher nach dem Einsetzen von natürlichem Knochenmaterial durchsetzt werden kann.

Weiter haben die Erfinder herausgefunden, dass der Zuschlag einer hydrophilen Komponente, insbesondere der Zuschlag von Wasser, das E-Modul reduziert und gleichzeitig die Streckgrenze bis zum Bruch erhöht. Dadurch kann in der Vertebroplastie und Kyphoplastie oder artverwandten Anwendungen gleichzeitig die Gefahr von Frakturen sowohl des ausgehärteten Knochenzements als auch des angrenzenden Knochenmaterials reduziert werden.

Die biodegradierbaren Partikel sind vorzugsweise in der hydrophilen Komponente enthalten. Theoretisch wäre aber auch denkbar, dass biodegradierbare Partikel zusammen mit einem Acryl-basierten Knochenzementpulver oder sogar mit dem Acrylatmonomer zugegeben werden, da die biodegradierbaren Partikel schon allein aufgrund ihrer Eigenschaften überwiegend in die hydrophile Komponente wandern würden.

Die hydrophile Komponente umfasst vorzugsweise Wasser sowie ein Calciumkarbonat, -sulfat und/oder -phosphat. Karbonat, Sulfat, Phosphat werden dabei im weitesten Sinne verstanden, also sämtliche Verbindungen von Schwefel, Phosphor und Karbonaten mit Calcium.

Insbesondere umfasst die hydrophile Komponente Hydroxylapatit. Das biodegradierbare Material, insbesondere das Hydroxylapatit, liegt in nanopartikulärer Form, also mit einer mittleren Partikelgröße von unter 100 nm, vor. Bei Verwendung von Stabilisatoren können auch größere Partikel, insbesondere mit einer Größe bis 20 µm verwendet werden.

Die Partikel liegen vorzugsweise in einer Suspension vor, welche beim Mischen mit der hydrophoben Komponente überwiegend zusammenhaftet und dabei Fadenähnliche Gebilde bildet, die zu einer interkonnektierten Struktur führt. Diese Eigenschaften hat eine zähflüssige Suspension aus Nanopartikeln in besonderem Maße.

Insbesondere nanopartikuläres Hydroxylapatit ist zudem in besonderer Weise geeignet, um das Einwachsen von Knochengewebe zu beschleunigen.

Derartiges Hydroxylapatit sowie dessen Herstellung ist beispielsweise in den europäischen Patentschriften EP 0 664 133 B1, EP 0 938 449 B1 und EP 1 317 261 B1 beschrieben. Im Hinblick auf das zugesetzte partikuläre Hydroxylapatit wird vollumfänglich auf den Offenbarungsgehalt dieser Dokumente verwiesen. Es werden insbesondere Hydroxylapatitpartikel beziehungsweise Suspensionen mit Hydroxylapatitpartikeln verwendet, wie sie in einem der genannten Dokumente beschrieben sind.

Die hydrophile Komponente enthält bei einer Weiterbildung der Erfindung einen eine Polymerisation der hydrophoben Komponente startenden Stoff, insbesondere einen Initiator. So kann der Knochenzement als Zwei-Komponenten-System bereitgestellt werden, bei welchem der Initiator in der hydrophilen Komponente enthalten ist. Als Initiator kann insbesondere Di-Benzoyl-Peroxid verwendet werden.

Bei einer bevorzugten Ausführungsform der Erfindung enthält die hydrophobe Komponente Polymerpartikel. Üblicherweise wird Knochenzement als eine Kombination von Monomer und Pulver bereit gestellt. So kann der angemischte Knochenzement mit pastenartiger Konsistenz bereitgestellt werden. Die Polymerpartikel werden beim Aushärten in der Regel angelöst und in der sich bildenden Polymermatrix eingebunden. Dadurch, dass nicht das vollständige Volumen polymerisieren muss, wird so auch die Erwärmung beim Aushärten reduziert.

Auch die hydrophile Komponente reduziert die Wärmeentwicklung beim Aushärten des erfindungsgemäßen Knochenzements, was einen zusätzlichen Vorteil des erfindungsgemäßen Knochenzements darstellt.

Bei einer Weiterbildung der Erfindung enthält der Knochenzement ein Röntgenkontrastmittel. Das Röntgenkontrastmittel kann dabei in der hydrophilen Komponente und/oder in der hydrophoben Komponente enthalten sein.

Bei einer weiteren Weiterbildung der Erfindung enthält der Knochenzement eine pharmazeutisch wirksame Substanz, insbesondere ein Antibiotikum. Das Antibiotikum ist vorzugsweise in der hydrophilen Komponente enthalten.

Bei einer bevorzugten Ausführungsform der Erfindung enthält der Knochenzement zwischen 10 und 50 % biodegradierbares Material, insbesondere biodegradierbare Partikel, zwischen 20 und 80% der polymerisierbaren hydrophoben Komponente, gegebenenfalls zwischen 10 und 60 % Polymerpartikel und zwischen 2 und 30 % Wasser (Angaben, soweit nicht anders angegeben, immer in Gewichts-%).

Die Erfindung betrifft des Weiteren einen Knochenzement, insbesondere mit einem oder mehrerer vorstehender Merkmale, welche eine Polymerkomponente sowie eine Flüssigkeit umfasst, welche mit der Polymerkomponente nicht mischbar ist. Insbesondere handelt es sich bei der Flüssigkeit um Wasser. Gemäß der Erfindung sind in der mit der Polymerkomponente nicht mischbaren Flüssigkeit biodegradierbare Partikel enthalten. Insbesondere liegen die biodegradierbaren Partikel als Suspension im Wasser vor. Bei der Polymerkomponente handelt es sich insbesondere um ein Monomer oder ein Prepolymer, welches zu einer Polymermatrix aushärtet. Um das Aushärten zu beschleunigen und die Wärmeentwicklung zu reduzieren, umfasst die Polymerkomponente üblicherweise auch Polymerpartikel, welche beim Anmischen mit dem Monomer gemischt werden.

Insbesondere handelt es sich um einen acrylatbasierten Knochenzement. Die Polymerkomponente ist mit der Suspension, in welcher die biodegradierbaren Partikel enthalten sind, nicht mischbar. Dadurch werden durch den Einschluss von Tropfen der Suspension Poren gebildet, in welchen die biodegradierbaren Partikel frei zugänglich sind.

Es hat sich herausgestellt, dass sich bei der Porenbildung zumindest teilweise langgezogene, kanalförmige Aussparungen bilden, so dass sich eine Polymermatrix mit interkonnektierenden Poren bildet.

Die Erfindung betrifft des Weiteren einen Knochenzement, umfassend 25 bis 80 % polymerisches Knochenzementpulver, 5 bis 30 % Wasser, 10 bis 70 % biodegradierbare Partikel sowie ein Monomer und einen Initiator.

Die Erfindung betrifft des Weiteren einen ausgehärteten Knochenzement, insbesondere einen Knochenzement, welcher aus dem vorstehend beschriebenen Knochenzement ausgehärtet ist. Der ausgehärtete Knochenzement umfasst ein Polymergerüst mit offenen Poren, welche zumindest teilweise mit biodegradierbarem Material gefüllt sind. Insbesondere handelt es sich um ein ausgehärteten Acrylat-basierten Knochenzement, dessen Polymergerüst ein Acrylat, insbesondere ein Polymethylmethacrylat umfasst.

Durch die Erfindung lässt sich insbesondere ein Acrylatbasierter Knochenzement bereitstellen, welcher im ausgehärteten Zustand ein E-Modul von unter 4500 MPa, vorzugsweise unter 2000 MPa und besonders bevorzugt unter 1600 MPa aufweist. Das E-Modul im Sinne der Erfindung wird im initial ausgehärteten Zustand nach einer Aushärtezeit von etwa fünf Stunden unter Anwendung der ISO5833:2002 Annex F gemessen. Mit dem erfindungsgemäßen Knochenzement kann über dass Verhältnis von hydrophober und hydrophiler Komponente die gewünschte Steifigkeit eingestellt werden.

Der erfindungsgemäße Knochenzement ist somit für einen Acrylat-basierten Knochenzement relativ weich, was die Gefahr von Folgefrakturen nach der Versorgung von Wirbelkörpern verringert.

Bei einer bevorzugten Ausführungsform weist der ausgehärtete Knochenzement bezogen auf das Polymergerüst eine Porosität zwischen 5 und 90, vorzugsweise zwischen 10 und 50 und besonders bevorzugt zwischen 10 und 35 % auf. Unter der Porosität im Sinne der Anmeldung wird die errechnete Porosität verstanden, bei welcher auch geschlossene Poren mit in die Porosität eingehen. Die hydrophile Komponente und das biodegradierbare Material, welches sich in der hydrophilen Komponente befindet, geht dagegen nicht in die Porosität ein.

Unter Porosität wird im Sinne der Erfindung insbesondere die errechnete Porosität verstanden, bei welcher das Volumen der hydrophoben Komponente, aus der sich das Polymergerüst ausbildet, mit dem Volumen der hydrophilen Komponente in Verhältnis gesetzt wird.

Bei einer Ausführungsform der Erfindung hat der ausgehärtete Knochenzement eine mittlere Porengröße zwischen 5 µm und 5 mm, vorzugsweise zwischen 20 und 200 µm. Die Porengröße kann u.a. durch Wahl des Mischverfahrens gesteuert werden. Auch ist denkbar, die Porengröße durch Zusätze, welche die Oberflächenspannung von Wasser reduzieren, zu verringern.

Bei einer Weiterbildung der Erfindung besteht das Polymergerüst zumindest teilweise aus einem quervernetzen Polymer. Insbesondere können zur Herstellung des Knochenzementes Polymerpartikel aus einem teilweise quervernetztem Polymer verwendet werden. Nach Zugabe des Monomers werden nicht quervernetzte Anteile angelöst und können als Bindeglied zu der sich als dem Monomer bildenden Polymermatrix dienen. So werden die Partikel auf molekularer Ebene vernetzt und nicht lediglich eingebettet.

Die Erfindung betrifft des Weiteren ein Verfahren zum Herstellen von Knochenzement, wobei eine polymerisierbare hydrophobe Komponente mit einer hydrophilen Komponente und biodegradierbarem Material vermischt wird.

Durch die hydrophile Komponente, welche sich mit der hydrophoben Komponente definitionsgemäß nicht mischt, bilden sich zumindest teilweise offenporige Bereiche, welche zunächst mit der hydrophilen Komponente ausgefüllt sind. Bei Verwendung des Knochenzements in vivo wird das eingeschlossene Wasser in der Regel durch Körperflüssigkeit ersetzt und die verbleibenden biodegradierbaren Partikel, welche in den Poren sitzen, sind frei zugänglich, was das Einwachsen des Knochenzements erheblich verbessert.

Das biodegradierbare Material wird vorzugsweise zusammen mit der hydrophilen Komponente als Suspension, insbesondere als Paste, zugemischt. Vorzugsweise ist die Paste hochviskos und im Wesentlichen formstabil, wie beispielsweise Quark oder Frischkäse. Die Viskosität kann insbesondere zwischen 1 und 100000 Pa·s betragen.

Bei Verwendung einer Suspension mit Nanopartikeln, die in der Suspension erzeugt worden sind, wie beispielsweise nanopartikuläres Hydroxylapatit, führen 20 bis 40 % Partikel in der Suspension zu der gewünschten Konsistenz.

Werden die Partikel als Pulver zugeführt oder werden ausgefällte Partikel verwendet, muss die Partikelkonzentration in der Regel höher sein, um die gewünschte Konsistenz zu erreichen. Gefälltes Material mit Partikeln kann daher einen Partikelanteil in der Suspension von 60 % oder mehr erforderlich machen.

Entscheidend ist, dass die hydrophile Komponente weder sedimentiert noch sich selbständig von der hydrophoben Komponente separiert. So bleibt eine zumindest teilweise straßenartige Struktur erhalten, die mit einer Partikelsuspension gefüllt ist.

Im Unterschied zu bekanntem Acryl-basiertem Hydroxylapatithaltigem Knochenzement sind die biodegradierbaren Partikel in hohem Maße zugänglich und fördern das Einwachsen des ausgehärteten Zementes.

Insbesondere ist vorgesehen, eine aufkonzentrierte Suspension mit calciumhaltigen Partikeln zu verwenden. Durch die Konsistenz der hydrophilen Komponente und/oder der hydrophoben Komponente sowie durch deren Verhältnis zueinander kann sowohl der Porositätsgrad als auch die Größe und in gewissen Grenzen auch das Aussehen der Poren, insbesondere der als Kanäle ausgebildeten Poren, bestimmt werden.

Vorzugsweise hat die als Suspension vorliegende hydrophile Komponente eine ähnliche Viskosität wie der restliche Knochenzement.

Über einen statistischen Mischer können die Komponenten miteinander vermischt werden, was insbesondere bei Verwendung zweier Pasten zu guten Ergebnissen führt.

Bei einer bevorzugten Ausführungsform der Erfindung ist dabei vorgesehen, dass beim Anmischen zumindest zwei Pasten verwendet werden, wobei eine erste Paste die polymerisierbare hydrophobe Komponente und die zweite Paste das biodegradierbare Material, einen Initiator und Wasser enthält.

Der erfindungsgemäße Knochenzement kann so als Zweikomponentensystem verwendet werden.

Vorzugsweise ist die erste Paste mit einem Accelerator versetzt, welcher die Polymerisation beschleunigt.

Als Accelerator wird insbesondere Dimethyl-p-Toluidin verwendet.

Die Verwendung eines vorpolymerisierten, teilweise quervernetztem Polymers, beispielsweise in Form eines Perlpolymerisats, führt nach dem Zumischen des Monomers zum Anlösen der Polymerpartikel und es wird die Bildung von langkettigen Polymeren aus dem Acrylatmonomer beschleunigt. Gleichzeitig können an- oder ausgelöste Bestandteile der Polymerpartikel zu einer Verdickung des Gemisches auf die gewünschte Viskosität führen.

Zur Stabilisierung des Monomers kann beispielsweise Hydrochinon zugesetzt werden, welches eine vorzeitige Polymerisation durch Wegfangen von Radikalen verhindert.

Bei einer Weiterbildung der Erfindung wird bei Herstellung der hydrophoben Komponente ein polymerisierbares Metallsalz verwendet. Insbesondere kann Zirkon(meth)- oder Barium(meth)acrylat verwendet werden. Aus dem Metallsalz wird vorzugsweise durch das Vorhandensein von Acrylsäure oder Methacrylsäure ein Metallacrylat, insbesondere ein Zirkon- oder Bariumacrylat gebildet.

Über ein Acrylat, insbesondere ein Metallacrylat, welches sich aufgrund der guten Abschirmung des Metallatoms gut in der hydrophoben Komponente lösen lässt, lassen sich Quervernetzungen bereit stellen. Es können sowohl Butylals auch Methylacrylate verwendet werden.

Insbesondere kann das Acrylat aus einem Acetat durch Zugabe von Methacrylsäure als ein partikuläres Prepolymer herstellt werden.

Das Acrylat kann insbesondere aus Zirkonacetat hergestellt werden. Das Acetat kann mittels Methacrylsäure ausgefällt werden und das entstandene Zirkonacrylat zur Herstellung der hydrophoben Komponente verwendet werden.

Auch andere Acrylate, wie beispielsweise Aluminiumacrylat, Magnesiumacrylat und Calciumacrylat können verwendet werden.

Insbesondere durch die Verwendung von Zirkon- oder Bariumacrylat kann dabei zugleich ein Röntgenkontrastmittel mit in die Polymermatrix eingebunden werden. Auch die Verwendung von Titanacrylat ist, insbesondere zur zusätzlichen Bereitstellung eines Röntgenkontrastmittels denkbar.

Ein Knochenzement kann beispielsweise wie folgt hergestellt werden.

### Beispiel 1

Als erster Schritt wird ein Acrylat- basierter Knochenzement aus einem Monomer und einem polymerischen Knochenzementpulver angemischt. Es entsteht dabei eine Knochenzementpaste, wie sie bereits nach dem Stand der Technik verwendet werden kann.

Nach Anmischung dieser Paste wird eine weitere Paste, bestehend aus einer Suspension aus fein dispers verteilten Partikeln aus Hydroxylapatit, Calciumkarbonat, Calciumsulfat und/oder Calciumphosphat angemischt und mit dem Knochenzement in einem geeigneten Mischgerät vermengt.

Die entstandene Paste aus den beiden Pasten kann nun verwendet werden.

Anstelle zunächst die Paste aus Acrylat-basiertem Knochenzement in einem getrennten Schritt herzustellen, kann eine aufkonzentrierte Suspension mit Hydroxylapatit, Calciumkarbonat, Calciumsulfat und/oder Calciumphosphat auch direkt mit den Polymerpartikeln des Acrylat-basierten Knochenzements und dem Monomer vermischt werden.

### Beispiel 2

In einem ersten Schritt wird eine Paste aus einer wässrigen Phase und fein dispers verteilten Partikeln aus Hydroxylapatit, Calciumkarbonat, Calciumsulfat und/oder Calciumphosphat sowie Di-Benzoyl-Peroxid mit einer zweiten Paste vermischt. Optional eine pharmakologisch wirksame Substanz. Die zweite Paste enthält Methyl(meth)acrylat und/oder Butylacrylat als Monomer, Di-Methyl-p-Toluidin als Accelerator sowie optional ein Röntgenkontrastmittel. Weiter enthält die zweite Paste ein teilweise quervernetztes PMMA-Perlpolymerisat. Die beiden Pasten werden mit einem geeigneten Mischer mit oder ohne statistischem Mischelement vermischt und können sodann mittels einer Spritze oder von Hand in Defektstellen eingebracht werden.

Durch das Methylmethacrylat löst sich das im Perlpolymerisat enthaltene PMMA teilweise aus den Kugeln heraus oder quillt die Kugeln an und verdickt das ansonsten sehr dünnflüssige Methylmethacrylat. Zur zusätzlichen Eindickung können noch weitere Polymerisate, insbesondere PMMA-Polymerisate, gelöste oder angequollene Polymere zugemischt werden.

### Beschreibung der Zeichnungen

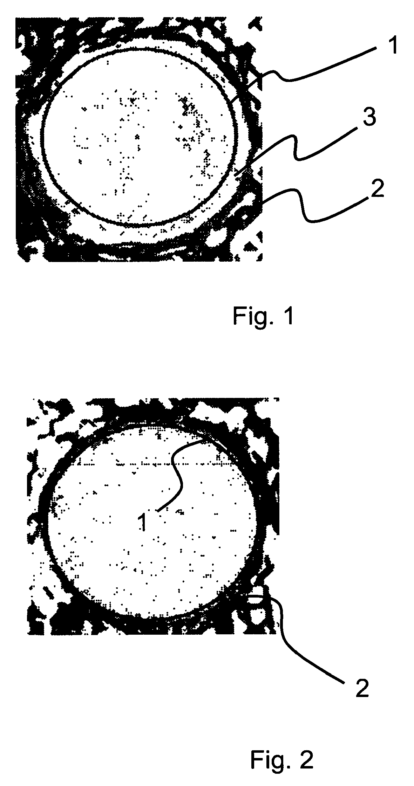

Die Erfindung soll im Folgenden bezugnehmend auf die Zeichnungen Fig. 1 bis Fig. 3 näher erläutert werden.

Fig. 1 und Fig. 2 zeigen schematisch den histologischen Befund der Femurkondyle eines Schafes, welcher wie folgt hergestellt wurde.

In den medialen Femurkondylen beider Hintergliedmaßen eines Schafes wurde durch eine Bohrung jeweils ein Defekt von etwa 15 cm Tiefe und 10 mm Durchmesser gesetzt und auf einer Seite mit herkömmlichem Acrylat-basiertem Knochenzement und auf der anderen Seite mit einem erfindungsgemäßen Knochenzement aufgefüllt. Die Euthanasie des Tieres fand drei Monate post operationem statt. Zur histologischen Untersuchung wurden die Kondylen in Scheiben gesägt und eingefärbte Knochenschliffe hergestellt.

Fig. 1 zeigt die mit herkömmlichem Acrylat-basierten Knochenzement gefüllte Defektstelle des Tieres, Fig. 2 zeigt die Defektstelle, welche mit einem Knochenzement gemäß eines Ausführungsbeispiels der Erfindung gefüllt wurde.

Um den Unterschied deutlicher sichtbar zu machen, wurden beide Aufnahmen mit einem gleich großen Ring 1 überlagert. Dieser Ring 1 könnte beispielsweise auch ein Implantat symbolisieren. Zu erkennen ist in Fig. 1, dass zwischen dem Ring 1 und dem natürlichen Knochenmaterial 2 ein Bereich 3 vorhanden ist, welcher allenfalls in geringem Maße von natürlichem Knochenmaterial durchsetzt ist. In Fig. 2 erstreckt sich das natürliche Knochenmaterial 2 dagegen bis zum Ring 1.

Es versteht sich, dass dieses Beispiel nur der Illustration des Effektes des erfindungsgemäßen Knochenzementes dient. In Detailaufnahmen ist deutlich zu erkennen, dass das natürliche Knochenmaterial deutlich von der Seite her in den Knochenzement einwächst.

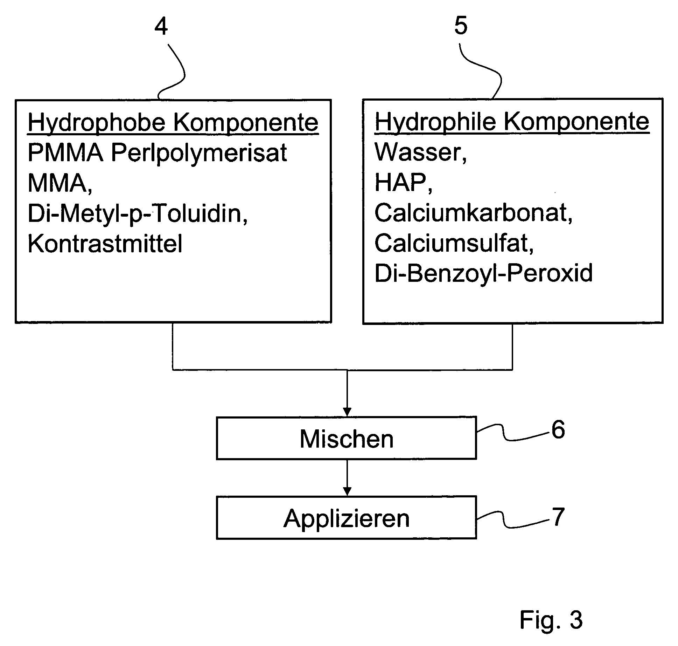

Fig. 3 zeigt ein Flussdiagramm eines schematisch dargestellten Herstellungs- bzw. Anmischverfahrens.

In diesem Ausführungsbeispiel wird der Knochenzement aus zwei Komponenten, einer hydrophoben Komponente 4 und einer hydrophilen Komponente 5 angemischt.

Die hydrophobe Komponente umfasst ein PMMA-Perlpolymerisat, Methylmethacrylat, Dimethyl-p-Toluidin als Accelerator sowie ein Kontrastmittel.

Die hydrophile Komponente umfasst eine wässrige Suspension mit Hydroxylapatit, Calciumkarbonat, Calciumsulfat und umfasst des Weiteren Di-Benzoyl-Peroxid, welches als Initiator der Polymerisation zugegeben wird.

Die beiden Komponenten werden in einem statistischen Mischer gemischt und können sodann appliziert werden.

Durch die Erfindung konnten die Eigenschaften von Acryl-basiertem Knochenzement dem jeweiligen Verwendungszweck entsprechend erheblich verbessert werden.

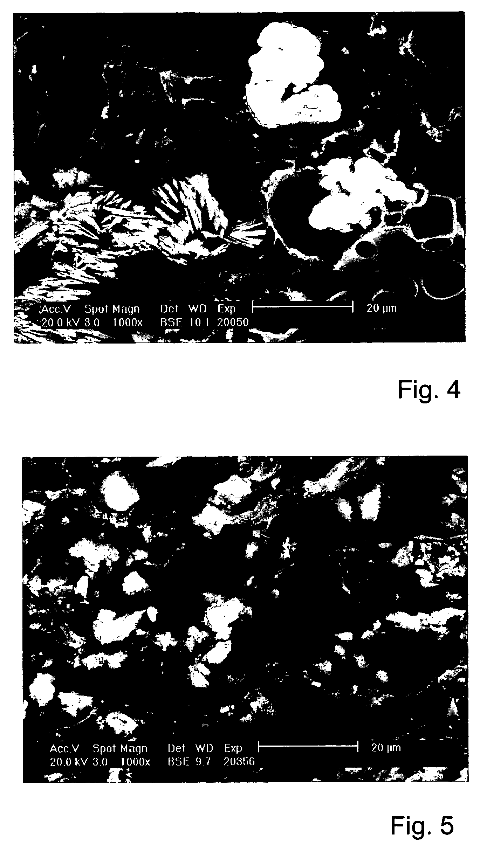

Fig. 4 bis 6 zeigen REM-Aufnahmen eines ausgehärteten erfindungsgemäßen Knochenzements.

In Fig. 4 und Fig. 5 ist gut zu erkennen, dass die hellen Partikel aus Hydroxylapatit, bei welchem es sich insbesondere um teilweise aneinanderhängende nanokristalline Strukturen handelt, nicht in der Polymermatrix eingebunden bzw. eingeklebt sind, sondern dass die Hydroxylapatitpartikel frei zugänglich in den Poren vorliegen. Insbesondere ist zu erkennten, dass die Partikel nicht dicht von der Polymermatrix umschlossen sind, sondern in der Regel nur ein Teil der jeweiligen Pore ausfüllen.

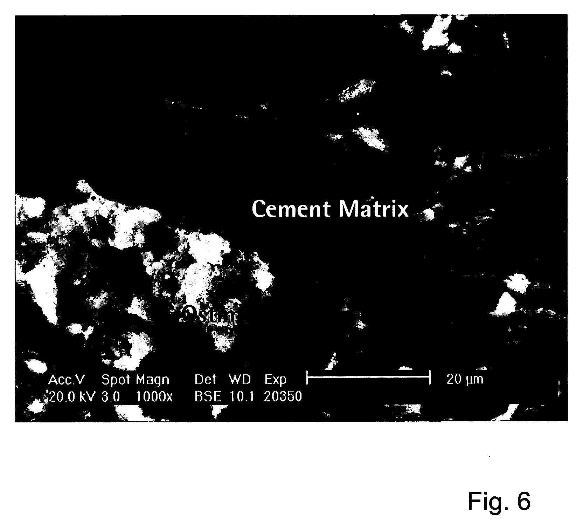

Fig. 6 zeigt eine weitere REM-Aufnahme, in welcher die Polymermatrix mit der Beschriftung "Zementmatrix" und ein Hydroxylapatitpartikel mit der Beschriftung "Ostim" versehen ist.

Es versteht sich, dass die Erfindung nicht auf eine Kombination vorstehend beschriebener Merkmale beschränkt ist, sondern dass der Fachmann sämtliche Merkmale, soweit dies technisch sinnvoll ist, kombinieren wird.

## Patentansprüche

1. Knochenzement, umfassend zumindest eine polymerisierbare hydrophobe Komponente, zumindest eine hydrophile Komponente und biodegradierbares Material, welches biodegradierbare Partikel umfasst, wobei sich hydrophile und hydrophobe Komponente sich nicht mischen und wobei die biodegradierbaren Partikel zumindest teilweise in der hydrophilen Komponente enthalten sind,
**dadurch gekennzeichnet, dass** hydrophile Komponente zusammen mit dem biodegradierbaren Material als Paste ausgebildet ist und wobei das biodegradierbare Material in nanopartikulärer Form vorliegt.

2. Knochenzement nach dem vorstehenden Anspruch, **dadurch gekennzeichnet, dass** zumindest 50 % des biodegradierbaren Materials in der hydrophilen Komponente enthalten sind.

3. Knochenzement nach einem der vorstehenden Ansprüche, **dadurch gekennzeichnet, dass** die hydrophobe Komponente ein Acrylatmonomer umfasst.

4. Knochenzement nach einem der vorstehenden Ansprüche, **dadurch gekennzeichnet, dass** die hydrophile Komponente Wasser sowie ein Calciumkarbonat, -sulfat und/oder -phosphat.

5. Knochenzement nach dem vorstehenden Anspruch, **dadurch gekennzeichnet, dass** die hydrophile Komponente Hydroxylapatit umfasst.

6. Knochenzement nach einem der vorstehenden Ansprüche, **dadurch gekennzeichnet, dass** die hydrophile Komponente ein eine Polymerisation der hydrophoben Komponente startendes System, insbesondere einen Initiator und einen Accelerator, umfasst und/oder dass die hydrophobe Komponente Polymerpartikel umfasst.

7. Knochenzement nach einem der vorstehenden Ansprüche, **dadurch gekennzeichnet, dass** der Knochenzement ein Röntgenkontrastmittel enthält und/oder dass der Knochenzement eine pharmazeutisch wirksame Substanz, insbesondere ein Antibiotikum, enthält und/oder dass der Knochenzement zwischen 10 und 50 % biodegradierbares Material, zwischen 10 und 85 % der polymerisierbaren hydrophoben Komponente und/oder zwischen 2 und 30 % hydrophile Komponente, enthält.

8. Knochenzement nach einem der vorstehenden Ansprüche, umfassend 10 bis 50 % polymerisches Knochenzementpulver, 10 bis 85 % polare Flüssigkeit, insbesondere Wasser, 10 bis 70 % biodegradierbare Partikel sowie ein Monomer und einen Initiator.

9. Ausgehärteter Knochenzement aus Knochenzement nach einem der vorstehenden Ansprüche, umfassend ein Polymergerüst mit offenen Poren, welche zumindest teilweise mit biodegradierbarem Material gefüllt sind.

10. Ausgehärteter Knochenzement nach dem vorstehenden Anspruch, **dadurch gekennzeichnet, dass** das Polymergerüst ein Acrylat, insbesondere Polymethymethacrylat, umfasst und/oder dass der ausgehärtete Knochenzement ein E-Modul von unter 4500 MPa, vorzugsweise unter 2000 MPa und besonders bevorzugt unter 1600 MPa aufweist und/oder dass der ausgehärtete Knochenzement bezogen auf das Polymergerüst eine Porosität zwischen 5 und 90, vorzugsweise zwischen 10 und 50% aufweist und/oder dass der der ausgehärtete Knochenzement eine mittlere Porengröße zwischen 5 µm und 5 mm, vorzugsweise zwischen 20 µm und 200 µm aufweist und/oder dass das Polymergerüst zumindest teilweise aus einem quervernetzen Polymer besteht und/oder dass der ausgehärtete Knochenzement offenporig pöros ist und in den Poren biodegradierbare Partikel angeordnet sind.

11. Verfahren zum Herstellen von Knochenzement, wobei eine polymerisierbare hydrophobe Komponente mit einer hydrophilen Komponente und biodegradierbarem Material vermischt wird, so dass sich durch die hydrophile Komponente zumindest teilweise offenporige Bereiche bilden, welche zunächst mit der hydrophilen Komponente ausgefüllt sind, **dadurch gekennzeichnet, dass** biodegradierbares Material und hydrophile Komponente als Paste zugemischt werden und wobei das biodegradierbare Material in nanopartikulärer Form vorliegt.

12. Verfahren zum Herstellen von Knochenzement nach dem vorstehenden Anspruch, **dadurch gekennzeichnet, dass** biodegradierbares Material in Form einer nanopartikulären Suspension und/oder einer einen Dispergator enthaltenden Suspension zugeführt wird und/oder dass als biodegradierbares Material ein Calciumkarbonat, -sulfat und/oder -phosphat zugemischt wird und/oder dass beim Anmischen zumindest zwei Pasten verwendet werden, wobei eine erste Paste die polymerisierbare hydrophobe Komponente und eine zweite Paste das biodegradierbare Material und einen Initiator enthält und/oder dass eine erste Paste mit einem Accelerator verwendet wird und/oder dass eine erste Paste mit Polymerpartikeln verwendet wird und/oder dass zur Herstellung der hydrophoben Komponente ein Acrylat verwendet wird, insbesondere dass das Acrylat mittels (Meth)acrylsäure ausgefällt wird und das entstandene Pulver zur Herstellung der hydrophoben Komponente verwendet wird und/oder dass ein Metallacrylsalz, insbesondere ein Zirkon-, Barium- oder Titanacrylsalz verwendet wird.

## Claims

1. A bone cement, comprising at least one polymerizable hydrophobic component, at least one hydrophilic component and biodegradable material, which comprises biodegradable particles, wherein said hydrophilic and said hydrophobic components are immiscible, and wherein said biodegradable particles are contained at least partially in the hydrophilic component,
**characterized in that** the hydrophilic component is embodied, together with the biodegradable material, as a paste, and wherein said biodegradable material is present in nanoparticulate form.

2. The bone cement of the preceding claim, wherein at least 50% of the biodegradable material are contained in said hydrophilic component.

3. The bone cement of any of the preceding claims, wherein said hydrophobic component comprises an acrylate monomer.

4. The bone cement of any of the preceding claims, wherein said hydrophilic component comprises water and at least one of calcium carbonate, calcium sulfate, and/or calcium phosphate.

5. The bone cement of any of the preceding claims, wherein the hydrophilic component comprises hydroxyapatite.

6. The bone cement of any of the preceding claims, wherein said hydrophilic component comprises a system that starts a polymerization of the hydrophobic component, in particular an initiator and an accelerator and/or wherein said hydrophobic component comprises polymer particles.

7. The bone cement of any of the preceding claims, further comprising an x-ray contrast agent and/or comprising a pharmaceutically active substance, in particular an antibiotic substance and/or wherein the bone cement comprises between 10% and 50% of said biodegradable particles, between 10% and 85% of said polymerizable hydrophobic component, and/or between 2 and 30% of said hydrophilic component.

8. The bone cement of any of the preceding claims, comprising 10 to 50% polymeric bone cement powder, 10 to 85 % polar liquid, in particular water, 10 to 70 % biodegradable particles and a monomer and an initiator.

9. A cured bone cement derived from a bone cement of any of the preceding claims, and further comprising a polymer backbone having open pores that are filled at least partially with biodegradable material.

10. The cured bone cement of the preceding claim, wherein said polymer backbone comprises an acrylate, in particular polymethyl methacrylate, and/or wherein the cured bone cement has an E-modulus of less than 4500 MPa, preferably of less than 2000 MPa, in particular preferred of less than 1600 MPa, and/or wherein cured bone the cured bone cement has, with respect to the polymer backbone, a porosity of between 5% and 90%, preferably between 10 and 50% and/or wherein the cured bone cement has an average pore size of between 5 µm and 5 mm, preferably between 20 µm and 200 µm and/or wherein said polymer backbone at least partially comprises a cross-linked polymer and/or wherein the cured bone cement is open-pore porous, and said biodegradable particles are in said open-pores.

11. A method for producing a bone cement, comprising the step of mixing a polymerizable hydrophobic component with a hydrophilic component and a biodegradable material, so that said hydrophilic component forms at least partially open-pore regions, which are first filled with the hydrophilic component,
**characterized in that** the biodegradable material and the hydrophilic component are mixed as a paste, and wherein said biodegradable material is present in nanoparticulate form.

12. The method of producing bone cement according to the preceding claim, further comprising the step of adding said biodegradable material in the form of a nanoparticulate suspension and/or a suspension containing a dispersant and/or a calcium carbonate, a calcium sulfate, and/or a calcium phosphate is admixed as biodegrable material
and/or wherein at least two pastes are used for mixing, wherein a first paste contains the polymerizable hydrophobic component and a second paste contains the biodegradable material and an initiator
and/or wherein a first paste comprises an accelerator and/or wherein a first paste comprises polymer particles and/or an acrylate is used for producing said hydrophobic component, in particular wherein said acrylate is precipitated with (meth)acrylic acid, and a powder that forms during said precipitating step is used for producing said hydrophobic component and/or wherein said acrylate is a metal acrylic salt, in particular a zircon acrylic salt, barium acrylic salt or titanium acrylic salt.

## Revendications

1. Ciment osseux, comprenant au moins un composant hydrophobe polymérisable, au moins un composant hydrophile et un matériau biodégradable, qui comprend des particules biodégradables, les composants hydrophiles et hydrophobes ne se mélangeant pas et les particules biodégradables étant présentes au moins partiellement dans le composant hydrophile, **caractérisé en ce que** le composant hydrophile prend la configuration d'une pâte conjointement avec le matériau biodégradable, et le matériau biodégradable se présentant sous la forme de nanoparticules.

2. Ciment osseux selon la revendication précédente, **caractérisé en ce qu'**au moins 50 % du matériau biodégradable sont présents dans le composant hydrophile.

3. Ciment osseux selon l'une des revendications précédentes, **caractérisé en ce que** le composant hydrophobe comprend un monomère d'acrylate.

4. Ciment osseux selon l'une des revendications précédentes, **caractérisé en ce que** le composant hydrophile comprend de l'eau ainsi qu'un carbonate, sulfate et/ou phosphate de calcium.

5. Ciment osseux selon la revendication précédente, **caractérisé en ce que** le composant hydrophile comprend de l'hydroxylapatite.

6. Ciment osseux selon l'une des revendications précédentes, **caractérisé en ce que** le composant hydrophile comprend un système déclenchant la polymérisation du composant hydrophobe, en particulier un initiateur et un accélérateur, et/ou **en ce que** le composant hydrophobe comprend des particules polymères.

7. Ciment osseux selon l'une des revendications précédentes, le ciment osseux étant **caractérisé en ce qu'**il contient un agent de contraste radiologique et/ou qu'il contient une substance pharmaceutiquement efficace, en particulier un antibiotique, et/ou **en ce qu'**il contient entre 10 et 50 % de matériau biodégradable, entre 10 et 85 % du composant hydrophobe polymérisable et/ou entre 2 et 30 % du composant hydrophile.

8. Ciment osseux selon l'une des revendications précédentes, comprenant 10 à 50 % de poudre de ciment osseux polymère, 10 à 85 % d'un liquide polaire, notamment de l'eau, 10 à 70 % de particules biodégradables ainsi qu'un monomère et un initiateur.

9. Ciment osseux durci constitué d'un ciment osseux selon l'une des revendications précédentes, comprenant un squelette polymère ayant des pores ouverts qui sont au moins partiellement remplis de matériau biodégradable.

10. Ciment osseux durci selon la revendication précédente, **caractérisé en ce que** le squelette polymère comprend un acrylate, en particulier un polyméthacrylate de méthyle, et/ou **en ce que** le ciment osseux durci présente un module E inférieur à 4500 MPa, de préférence inférieur à 2000 MPa et particulièrement de préférence inférieur à 1600 MPa et/ou **en ce que** le ciment osseux durci présente, par rapport au squelette polymère, une porosité située entre 5 et 90, de préférence entre 10 et 50 % et/ou **en ce que** le ciment osseux durci présente une taille moyenne des pores située entre 5 µm et 5 mm, de préférence entre 20 µm et 200 µm et/ou **en ce que** le squelette polymère se compose au moins partiellement d'un polymère à réticulation croisée et/ou **en ce que** le ciment osseux durci est poreux avec des pores ouverts et **en ce que** des particules biodégradables sont disposées dans les pores.

11. Procédé de fabrication d'un ciment osseux, dans lequel un composant hydrophobe polymérisable comprenant un composant hydrophile et un matériau biodégradable est mélangé de telle sorte que le composant hydrophile permet de former des zones ayant au moins partiellement des pores ouverts, remplies au préalable de composant hydrophile, **caractérisé en ce que** le matériau biodégradable et le composant hydrophile sont ajoutés sous forme de pâte et le matériau biodégradable se présente sous la forme de nanoparticules.

12. Procédé de fabrication d'un ciment osseux selon la revendication précédente, **caractérisé en ce que** le matériau biodégradable est amené sous la forme d'une suspension nanoparticulaire et/ou d'une suspension contenant un agent dispersant et/ou **en ce qu'**un carbonate, sulfate et/ou phosphate de calcium est ajouté comme matériau biodégradable et/ou **en ce qu'**au moins deux pâtes sont utilisées lors du mélange, une première pâte contenant le composant hydrophobe polymérisable et une deuxième pâte contenant le matériau biodégradable et un initiateur et/ou en ce qu'une première pâte comprenant un accélérateur est utilisée et/ou **en ce qu'**une première pâte comprenant des particules polymères est utilisée et/ou **en ce qu'**un acrylate est utilisé pour la préparation du composant hydrophobe, notamment **en ce que** l'acrylate est recristallisé au moyen d'acide (méth)acrylique et **en ce que** la poudre obtenue pour la préparation du composant hydrophobe est utilisée et/ou **en ce qu'**un sel acrylique métallique, en particulier un sel acrylique de zirconium, de baryum ou de titane, est utilisé.
